# NEW EUROPEAN PATENT SPECIFICATION

(11) **EP 0 176 511 B2**
(45) Date of publication and mention of the opposition decision: **14.04.1993**
(45) Mention of the grant of the patent: 07.09.1988
(21) Application number: 84902234.8
(22) Date of filing: 21.05.1984
(51) Int. Cl.: A61J 1/00

(54) **A PRESSURE BALANCING DEVICE FOR SEALED VESSELS**
DRUCKAUSGLEICHVORRICHTUNG FÜR VERSIEGELTE BEHÄLTER
DISPOSITIF D'EQUILIBRAGE DE PRESSION POUR RECIPIENTS SCELLES

(30) Priority: 20.05.1983 SE 8301176; 02.03.1984 WO PCT/SE84/00075
(43) Date of publication of application: 09.04.1986
(73) Proprietor: Gustavsson, Bengt, S-421 79 Västra Frölunda (SE)
(72) Inventor: Gustavsson, Bengt, S-421 79 Västra Frölunda (SE)
(74) Representative: Roth, Ernst Adolf Michael
(86) International application number: PCT/SE84/00195
(87) International publication number: WO 84/04672

(56) References cited:
- EP-A- 0 091 312
- EP-A- 0 123 659
- FR-A- 2 221 121
- SE-B- 341 982
- US-A- 4 161 178

## Description

### Technical field

The present invention refers to a device for ventilating and pressure balancing the interior of a sealed vessel containing a substance which is to be taken out from said vessel e.g. by an injection syringe, said vessel having a neck fitted with a sealing member through which a puncturing member, e.g. needle can be passed for entering the interior of said vessel.

### Background of the invention

Drugs and medicines of different kinds which are to be injected into a patient are usually delivered in vials sealed by a membrane or sealing plug which can be penetrated by the needle of an injection syringe. The substances are in some cases delivered as solutions and in other cases as dry substances, which firstly have to dissolved in a solvent, e.g. water, which is injected into the vial by an injection syringe. In both cases there are pressure balancing problems when pressing liquid into or taking out from the air-tightly sealed vial.

In GB-A-1.461.161 there is disclosed a device for preparing a solution from a dry ingredient and a solvent contained in the bottle. The device comprises two interconnected elastically deformable containers, one containing the dry ingredient and the other being air-filled. The containers are provided with hollow needles penetrating the stopper of the bottle. The air filled container is arranged for creating an overpressure in the bottle, so that solvent is pressed into the container containing the dry ingredient.

EP-A-0 123 659 which was published after the priority date of the present application and, therefore falls under Article 54 (3) and (4) EPC discloses an expandable bag communicating with the interior of an ampoule through a cannula penetrating the stopper of the ampoule.

### The object of the invention and its most important features

The object of the present invention is to provide a pressure balancing system especially intended for vials containing toxic substances, such as cytotoxic drugs, etc. which are not allowed to contaminate the ambient air. This has according to the invention been achieved by the fact that said device comprises a closed container and connection means in the form of a cap to which the closed container is firmly attached and which is provided with attachment means for firmly attaching the device to the neck portion of said vessel and further, at its opposite portion, which in use position is facing away from said vessel, with coupling means for said transfer member and with a pierceable sealing member ovelying the-sealing member of the vessel in use position of said device, said connection means further having an internal passage for ventilating the vessel, said passage communicating with said closed container by way a liquid-rejecting filter arranged to prevent entry of liquid into the closed container and, in use position of said device, also communicating with the vessel, either directly or via t he puncturing memberof the transfer device, to provide a communication between the closed container and the interior of the vessel in use position of said device for ventilating and pressure balancing the interior of the vessel.

### Brief description of the drawings

Fig. 1 is a longitudinal section of a closure means to a vial according to the present invention.
Fig. 2 is a section according to the line II-II in fig. 3.
Fig. 3-4 are longitudinal sections through two further embodiments.

### Description of some embodiments

Referring to the drawings the numeral 1 denotes a vial sealed by a stopper 2. A connection means 3 is via attachment means 20 attached to the bottle-neck of the vial 1. The connection means 3 has a bayonet coupling 4 for receiving a transferring member (not shown) of the kind disclosed in the Swedish patent specification No. 8301176-7 (434 700) and International patent specification No. WO 84/04673 and for transferring the content of the vial to an injection syringe without any contamination risk. A sealing member 5, e.g. of Teflon (R), which can be penetrated by a needle is provided in the connection means 3, which further is provided with a hood 6 extending radially a considerable distance outside the bottle-neck of the vial 1. Atorus-shaped expandable balloon 7 is placed under the hood 6 and attached to a cylindrical balloon attachment 8 provided with a liquid-rejecting filter 9. The balloon 7 communicates with the interior of the vial 1 via a hollow needle 10 passed through the stopper 2. The balloon attachment 8 could of course be angled at which angling of the balloon 7 is avoided.

The balloon 7 works as a pressure equalizer when handling the contents of the vial 1. In case the vial 1 contains a dry substance this mustfirstly be dissolved in a solvent, e.g. water, which is injected with an injection syringe. This will create an overpressure in the vial 1 and the surplus air is then pressed into the balloon 7. On sucking up the dissolved substance into the injection syringe air is sucked back into the vial from the balloon and a negative pressure is avoided. A completely closed pressure equaliztion system has thus been achieved.

In inflated condition the walls of the balloon 7 are stretched and they exert a counter-pressure on the volume of air contained therein.

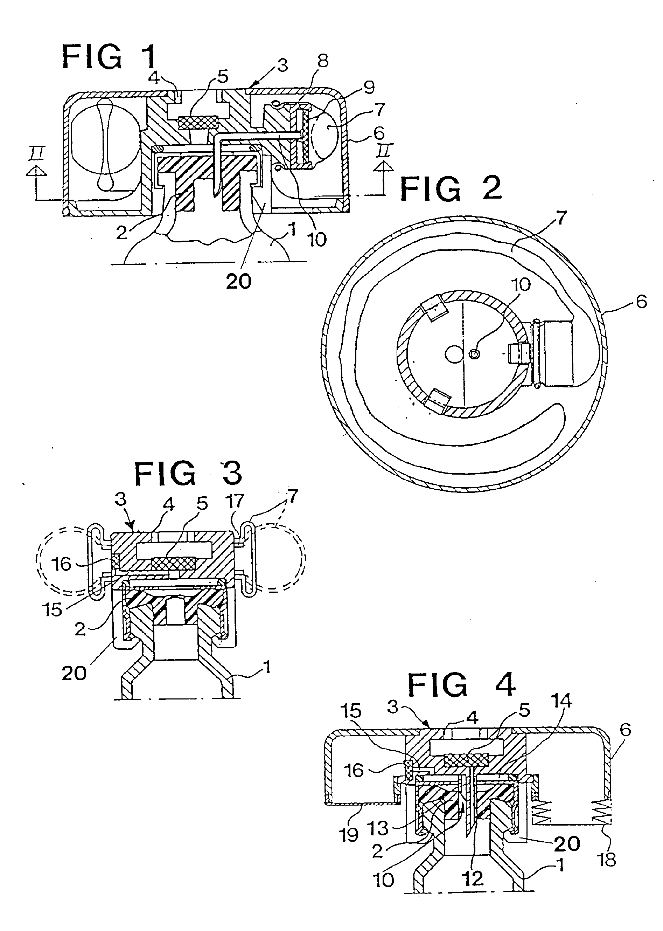

In fig. 3 is shown an embodiment in which communication between the interior of the vial 1 and the balloon 7 via an internal passage 15 in the connection means 3 can be provided by an additional needle penetrating the stopper 2 together with the needle (not shown) for transferring the substance to an injection syringe. Said additional needle can be designed in different ways as described in the International specification No. WO 84/04673.

The connection means 3 is further on its outside provided with a ring-shaped attachment 17 for a ring-shaped flexible member 7 inflatable like a balloon and arranged to cover the filter 16.

In fig. 4 is shown a combination of two furtherem- bodiments similar to the one according to fig. 1, but here according to one of the embodiments the flexible member communicating with the interior of the vial 1 via the hollow piercing member 10 consists of a bellows 18 instead of a balloon 7. The bellows 18 is attached to the lower edges of the hood 6 so that a closed space is provided under said hood. The bellows is pressure-less both in retracted and extended condition. Instead of a bellows a piston-cylinder or similar arrangement, the volume of which is variable, can be used. According to the other embodiment shown in fig. 4 the interior of the vessel communicates with a space closed by a lid 19 and the volume of which is constant. If the volume of said space is sufficient it can serve as a pressure-balancer or buffer for the vessel. The piercing member 10 is provided with two passages 12 and 13 one 12 for the needle (not shown) for transferring the substance and the other 13 for providing a communication between the interior of the vessel and the internal passage 15 via a space 14 between the vessel and the connection means 3, said internal passage 15 communicates with said pressure-balancing space underthe hood 6.

## Claims

1. A device intended to be used in combination with a vessel (1) having a neck fitted with a sealing member (2) and further intended to be able to receive a transfer member comprising a puncturing member, e.g. a needle of an injection syringe, which is able to pierce the sealing member (2) to enter the interior of the vessel for removing or adding material thereto, said device comprising a closed container (7;18;19) and connection means (3) in the form of a cap to which the closed container is firmly attached and which is provided with attachment means (20) for firmly attaching the device to the neck portion of said vessel (1) and further, at its opposite portion, which in use position is facing away from said vessel, with cou- piing means (4) for said transfer member and with h a pierceable sealing member (5) overlying the sealing member (2) of the vessel in use position of said device, said connection means (3) further having an internal passage (10;13;14;15) for ventilating the vessel, said passage communicating with said closed container (7;18;19) by way a liquid-rejecting filter (9;16) arranged to prevent entry of liquid into the closed container and, in use position of said device, also communicating with the vessel (1), either directly or via the puncturing member of the transfer device, to provide a communication between the closed container and the interior of the vessel in use position of said device for ventilating and pressure balancing the interior of the vessel.

2. A device according to claim 1,
characterized in,
that said container consists of a balloon (7) having extensible side walls and which in inflated condition exerts a counterpressure on the volume of air contained therein.

3. A device according to claim 1,
characterized in,
that said container consists of a bellows (18), piston-cylinder or the like, the volume of which is variable and which is pressure-less in both retracted and extended position.

4. A device according to claim 1,
characterized in,
that the volume of said container is constant.

5. A device according to any of the preceding claims,
characterized in,
that at least a part of said internal passage (10;13) is a bore of a hollow puncturing member of said device adapted to extend through said sealing member (2) in use position of said device.

## Patentansprüche

1. Eine in Verbindung mit einem am Hals mit einer Dichtung versehenen Gefäß (1), zu verwendende Vorrichtung, die darüber hinaus ein Übertragungselement mit Spitze, z.B. die Nadel einer Injektionsspritze, aufnehmen kann, mit der die Dichtung (2) durchstochen werden kann, um in das Innere des Gefäßes zu gelangen und Material einzufüllen bzw. zu entnehmen, wobei die genannte Vorrichtung einen geschlossenen Behälter (7;18;19) sowie einen kappenförmigen Verbinder (3) umfaßt, mit dem der geschlossene Behälter fest verbunden ist und der mit Befestigungselementen (20) versehen ist, mittels derer die Vorrichtung fest am Hals des genannten Gefäßes (1) gehalten wird sowie außerdem auf der gegenüberliegenden, in der Anwendungsposition von dem genannten Gefäß wegweisenden Seite, mit Kopplungselementen (4) für das genannte Übertragungselement und mit einer durchstechbaren, die Dichtung (2) des Gefäßes in der Anwendungsposition der genannten Vorrichtung überlagernden Dichtung (5), wobei der genannte Verbinder (3) zur Belüftung des Gefäßes über einen internen Kanal (10;13;14;15) verfügt, welcher mit dem genannten geschlossenen Gefäß (7;18;19) über ein flüssigkeitsabweisendesFilter (9;16) kommuniziert, das so angeordnet ist, daß das Eintreten von Flüssigkeit in den geschlossenen Behälter verhindert wird und in der Anwendungsposition der genannten Vorrichtung auch direkt oder über das Einstichteil des Übertragungselements mit dem Gefäß (1) verbunden ist, um eine Verbindung zwischen dem geschlossenen Behälter und dem Inneren des Gefäßes in derAnwendungsposition der genannten Vorrichtung zum Zwecke der Belüftung und des Druckausgleichs im Gefäßinneren zu schaffen.

2. Eine Vorrichtung gemäß Anspruch 1,
gekennzeichnet dadurch daß
der genannte Behälter aus einem Ballon (7) mit dehnbaren Seitenwänden besteht, der im aufgeblasenen Zustand einen Gegendruck auf das darin enthaltene Luftvolumen ausübt.

3. Eine Vorrichtung gemäß Anspruch 1,
gekennzeichnet dadurch daß
der genannte Behälter aus einem sowohl in eingezogenem als auch in ausgedehntem Zustand drucklosen Faltenbalg (18), Kolbenzylinder oder ähnlichem Gefäß mit veränderlichem Volumen besteht.

4. Eine Vorrichtung gemäß Anspruch 1,
gekennzeichnet dadurch daß das Volumen des genannten Behälters konstant ist.

5. Eine Vorrichtung gemäß einem der obengenannten Ansprüche,
gekennzeichnet dadurch daß
mindestens ein Teil des genannten inneren Kanals (10;13) eine Bohrung eines hohlen Einstichteils ist, welches so angepaßt wurde, daß es in der Anwendungsposition der genannten Vorrichtung durch die genannte Dichtung (2) hindurchführt.

## Revendications

1. Dispositif destiné à être utilisé en combinaison avec un récipient (1) comportant un col muni d'un élément d'étanchéité (2) et de plus destiné à pouvoir recevoir un moyen de transfert comprenant un élément de perforation, par exemple une aiguille d'un seringue pour injection, qui est capable de percer l'élément d'étanchéité (2) pour pénétrer à l'intérieur du récipient pour y retirer ou ajouter un produit, ledit dispositif comprenant un réservoir fermé (7, 18, 19) et un moyen de liaison (3) en forme de capot auquel le réservoir fermé est solidement attaché et qui est doté de moyens de fixation (20) pour solidement attacher le dispositif où la partie col dudit récipient (1) et de plus, à sa partie opposée, qui, en position d'utilisation, est située à l'opposé dudit récipient, de moyens d'accouplement (4) pour ledit moyen de transfert et d'un élément d'étanchéité perforable (5) recouvrant ledit élément d'étanchéité (2) du récipient en position d'utilisation dudit dispositif, ledit moyen de liaison (3) ayant en outre un passage interne (10, 13, 14, 15) pour aérer le récipient, ledit passage communiquant avec ledit réservoir fermé (7, 18, 19) par un filtre (9, 16) à rejet de liquide disposé pour empêcher toute entrée de liquide dans le réservoirfermé et, en position d'utilisation dudit dispositif, communiquant avec le récipient (1), soit directement, soit par l'élément de perforation du dispositif de transfert, pour assurer une communication entre le réservoirfermé et l'intérieur du récipient en position d'utilisation dudit dispositif pour aérer et équilibrer la pression à l'intérieur du récipient.

2. Dispositif selon la revendication 1, caractérisé par le fait que le réservoir est constitué par un ballon (7) présentant des parois latérales extensibles et qui, à l'état gonflé, excerce une contre- pression sur le volume d'air qui y est contenu.

3. Dispositif selon la revendication 1, caractérisé par le fait que le réservoir est constitué par un soufflet (18), un piston-cylindre ou similaire, dont le volume est variable et qui est non comprimé à la fois en position rétractée et en position étendue.

4. Dispositif selon la revendication 1, caractérisé par le fait que le volume du réservoir est constant.

5. Dispositif selon l'une quelconque des revendications précédentes, caractérisé par le fait qu'au moins une partie dudit passage interne (10, 13) est au calibre d'un élément de perforation creux dudit dispositif adapté pour traverser ledit élément d'étanchéité (2) en position d'utilisation dudit dispositif.
